# EUROPÄISCHE PATENTSCHRIFT

(11) **EP 1 013 634 B1**
(45) Veröffentlichungstag und Bekanntmachung des Hinweises auf die Patenterteilung: **21.05.2003**
(21) Anmeldenummer: 99124072.2
(22) Anmeldetag: 10.12.1999
(51) Int. Cl.: C07C 68/00, C07C 69/96

(54) **Verfahren zur Herstellung von Oligocarbonaten**
Process for the preparation of oligocarbonates
Procédé de préparation d'oligocarbonates

(30) Priorität: 22.12.1998 DE 19859289
(43) Veröffentlichungstag der Anmeldung: 28.06.2000
(73) Patentinhaber: BAYER AG, 51368 Leverkusen (DE)
(72) Erfinder: Reisinger, Claus-Peter, Dr., 47798 Krefeld (DE); Ebert, Wolfgang, Dr., 47800 Krefeld (DE)

(56) Entgegenhaltungen:
- EP-A- 0 749 955
- EP-A- 0 801 051

## Beschreibung

Es ist prinzipiell bekannt, organische Oligocarbonate durch oxidative Umsetzung einer aromatischen Dihydroxyverbindung mit Kohlenmonoxid in Gegenwart eines Edelmetall-Katalysators herzustellen (DE-OS 27 38 437, EP-A-0 801 051, EP-A-0 749 955); diese Oligocarbonate können anschließend zu Polycarbonat aufkondensiert werden (DE-OS 40 32 924). Als Edelmetall wird bevorzugt Palladium eingesetzt. Zusätzlich werden ein Cokatalysator (z.B. Mangan- oder Kobaltsalze), eine Base, ein quatemäres Salz, verschiedene Chinone bzw. Hydrochinone und Trockenmittel eingesetzt. Dabei kann in einem inerten Lösungsmittel, bevorzugt Methylenchlorid, gearbeitet werden (DE-OS 28 15 501).

Die Effizienz der beschriebenen Katalysatorsysteme ist aber gering; häufig wird das Edelmetall Palladium sogar stöchiometrisch eingesetzt. Die bisher höchste beschriebene Aktivität beläuft sich auf weniger als fünf Katalysezyklen pro Palladiumatom (DE-OS 28 15 501).

Es wurde nun überraschend gefunden, daß der Zusatz inerter organischer Lösungsmittel zum Reaktionsgemisch, die unter den Reaktionsbedingungen ein Azeotrop mit Wasser bilden, und die Entfernung dieses Azeotrops aus dem Reaktionsgemisch es ermöglicht, die Aktivität des Katalysatorsystems erheblich zu steigern.

Gegenstand der Erfindung ist somit ein Verfahren zur Herstellung eines aromatischen Oligocarbonats der Formel

HO-[A₁-Y-A₂-O-CO-O]ₙ-A₁-Y-A₂-OH (I),

wobei
- A₁, A₂: voneinander unabhängig divalente, gegebenenfalls verzweigte bzw. substituierte, carbocyclische, heterocyclische oder aromatische Gruppen repräsentieren,
- Y: eine divalente Gruppe wie Alkylen, Alkyliden, Cycloalkylen, Cycloalkyliden, -S-, -O-, -SO₂-, -SO- und -CO- oder eine Einfachbindung bedeutet und
- n: Zahlen von 1 bis 75 annehmen kann,
durch Umsetzung einer aromatischen Hydroxyverbindung der Formel

HO-A₁-Y-A₂-OH (II),

worin A₁, A₂ und Y die oben angegebenen Bedeutungen haben,
mit Kohlenmonoxid und Sauerstoff in Gegenwart eines Platinmetall-Katalysators, eines Cokatalysators, einer Base, und gegebenenfalls eines quaternären Salzes sowie eines inerten organischen Lösungsmittels, wobei das inerte organische Lösungsmittel unter den Reaktionsbedingungen ein Azeotrop mit dem bei der Reaktion entstehenden Wasser bildet, und dieses Azeotrop aus dem Reaktionsgemisch entfernt wird.

In einer bevorzugten Ausführungsform wird die Entfernung des Wassers aus dem Reaktionsgemisch als Azeotrop mit dem Lösemittel durch Strippen mit überschüssigen Reaktionsgas unterstützt. Dabei ist entscheidend, daß mehr als 5 Vol.-%, bevorzugt mehr als 10 Vol.-%, des Lösemittels in Form des Azeotrops aus der Reaktionsmischung entfernt werden. Unter reinen Strippbedingungen (DE-OS 44 03 075), d.h. ohne Azeotropbildung mit dem organischen Lösemittel und gleichzeitiger Entfernung des Azeotrops aus dem Reaktionsgemisch, werden deutlich geringere Umsätze erzielt.

Das erfindungsgemäße Verfahren zur Oligocarbonatbildung wird bei einer Reaktionstemperatur von 30 bis 200°C, bevorzugt 30 bis 150°C, besonders bevorzugt 60 bis 130°C, und bei einem Gesamtdruck von 1 bis 200 bar, bevorzugt 1 bis 60 bar, besonders bevorzugt 1 bis 20 bar durchgeführt.

Als inertes organisches Lösungsmittel können geeignet siedende, mit Wasser azeotropbildende halogenierte Kohlenwasserstoffe und aromatische Lösemittel wie Chlorbenzol, Dichlorbenzol, Fluorbenzol, Benzol, Anisol, Methylenchlorid oder 1,2-Dichlorethan, gegebenenfalls auch Mischungen davon, verwendet werden. Besonders bevorzugt wird Chlorbenzol eingesetzt. Das inerte Lösemittel kann mit einem Anteil von 1-99 Gew.-%, bevorzugt 20-98 Gew.-%, besonders bevorzugt 40-98 Gew.-% in der Reaktionsmischung enthalten sein.

Durch ein im azeotrophaltigen Abgasstrom befindliches Trennorgan, wie z.B. einen Dephlegmator, eine Destillationskolonne mit Böden oder Packung und weitere dem Fachmann bekannte Apparate, kann der größte Teil des bei der Entwässerung mitgerissenen Lösungsmittels vom Wasser abgetrennt und in den Rückstrom zum Reaktor geführt werden. Die Trennung bzw. Brechung des abgetrennten Azeotrops kann nach dem Stand der Technik z.B. durch Extraktion, Ausfrieren oder Destillation erfolgen.

Der mit dem Azeotrop ausgetriebene Anteil an gelösten Gasen läßt sich in einer bevorzugten Ausführungsform nach der Abtrennung wieder dem Reaktorkreisgas zuführen. Die Abtrennung mitgerissener Edukte, Lösungsmittel, Produkte und Wasser aus dem zu recyclisierenden gegebenenfalls vor der Trennung komprimierten Gasgemisch erfolgt nach dem Stand der Technik, z.B. durch Adsorption, Absorption oder bevorzugt durch Kondensation. Das zur Reaktion benötigte Reaktionsgas, bestehend aus Kohlenmonoxid, Sauerstoff und gegebenenfalls einem Inertgas, wird hierzu in einer Menge von 1 bis 10000 Nl pro Liter Reaktionslösung, bevorzugt 5 bis 5000 Nl pro Liter Reaktionslösung und besonders bevorzugt 10 bis 1000 Nl pro Liter Reaktionslösung eingeleitet. Das aus der Entwässerung stammende, zu recyclisierende Gasgemisch wird bezüglich seiner Anteile an CO und O₂ auf die genannten Volumina angerechnet.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,001-1,0) bevorzugt 1:(0,01- 0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig werden explosionsfähige Kohlenmonoxid/Sauerstoff-Gasgemische vermieden. Alle Ausgangsverbindungen können mit Verunreinigungen aus ihrer Herstellung und Lagerung kontaminiert sein, jedoch ist es im Sinne der Reinheit des Endproduktes wünschenswert, mit möglichst sauberen Chemikalien zu arbeiten. Auch die Reaktionsgase unterliegen keinen besonderen Reinheitsanforderungen. So kann Synthesegas als CO-Quelle und Luft als O₂-Träger dienen, es ist jedoch darauf zu achten, daß keine Katalysatorgifte wie z.B. Schwefel oder dessen Verbindungen eingetragen werden. In der bevorzugten Ausführungsform des erfindungsgemäßen Verfahrens werden reines CO und reiner Sauerstoff verwendet.

Die erfindungsgemäß umsetzbaren aromatischen Dihdroxyverbindungen können mit C₁-C₄-Alkyl, C₁-C₄-Alkoxy, Fluor, Chlor oder Brom substituiert sein. Bei den erfindungsgemäß umsetzbaren aromatischen Dihdroxyverbindungen handelt es sich beispielsweise um Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren kernalkylierte oder kernhalogenierte Verbindungen.

Bevorzugte Diphenole sind z.B. 2,2-Bis-(4-hydroxyphenyl)-propan (Bisphenol A), 2,4-Bis-(4-hydroxyphenyl)-2-methylbutan, 1,3-Bis-(4-hydroxycumyl)-benzol, 1,4-Bis-(4-hydroxycumyl)-benzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 1,3-Bis-(3,5-dimethyl-4-hydroxycumyl)-benzol, 1,4-Bis-(3,5-dimethyl-4-hydroxycumyl)-benzol, 1,1-Bis-(3,5-dimethyl-4-hydroxyphenyl)-2-methylbutan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan, 1,1-Bis(4-hydroxyphenyl)-1-phenyethan und 1,1-Bis-(4-hydroxyphenyl)-3,3,5-trimethylcyclohexan. Besonders bevorzugte Diphenole sind 4,4'-Dihydroxydiphenyl, 4,4'-Dihydroxydiphenylsulfid, 2,2-Bis-(4-hydroxyphenyl)-propan, 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,1-Bis(4-hydroxyphenyl)-1-phenyethan, 1,1-Bis-(4-hydroxyphenyl)-cyclohexan und 1,1-Bis-(4-hydroxyphenyl)-3,3,5-trimethylcyclohexan.

Die Oligocarbonate können durch den Einsatz geringer Mengen Verzweiger verzweigt werden. Beispiele für geeignete Verzweiger sind Phloroglucin, 4,6-Dimethyl-2,4,6-tri-(4-hydroxyphenyl)-hept-2-en, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl)-propan, 2,4-Bis-(4-hydroxyphenyl-isopropyl)-phenol, 2,6-Bis-(2-hydroxy-5'-methyl-benzyl)-4-methylphenol, 2-(4-Hydroxyphenyl)-2-(2,4-dihydroxyphenyl)-propan, Hexa-(4-(4-hydroxyphenyl-isopropyl)-phenyl)-orthoterephthalsäureester, Tetra-(4-hydroxyphenyl)-methan, Tetra-(4-(4-hydroxyphenyl-isopropyl)-phenoxy)-methan, 1,1-Bis-((4',4"-dihydroxytriphenyl)-methyl)-benzol und insbesondere α,α',α''-Tris-(4-hydroxyphenyl)-1,3,5-triisopropenylbenzol.

Die gegebenfalls mitzuverwendenden 0,05 bis 2 Mol% an Verzweigern bezogen auf eingesetzte Mole Diphenol, können entweder mit den Diphenolen selbst im organischen Lösemittel vorgelegt oder während der Reaktion zugegeben werden.

In das erfindungsgemäße Verfahren einsetzbare Basen sind Alkalihydroxide, Alkalisalze bzw. quaternäre Salze von schwachen Säuren wie Alkali-tert.-butylate. Es kann auch das Mono- oder Dialkalisalz bzw. die quaternären Salze der aromatischen Dihydroxyverbindung der allg. Formel (II) verwendet werden, welche zum organischen Oligocarbonat umgesetzt werden soll. Die Alkalisalze können Lithium-, Natrium-, Kalium-, Rubidium- oder Cäsiumsalze und die quaternären Salze können u.a. Tetraalkylammonium- oder Tetraalkylphosphoniumsalze sein. Bevorzugt werden Mono- und Dilithium-, Mono- und Dinatrium-, und Mono- und Dikaliumdiphenolate bzw. Lithium-, Natrium- oder Kalium-tert.-butylat, besonders bevorzugt das Mono- bzw. das Dikaliumdiphenolat der aromatischen Dihydroxyverbindung der allg. Formel (II) und Kalium-tert.-butylat eingesetzt.

Die Base wird in katalytischen Mengen zugesetzt Das Verhältnis von Platinmetall, z.B. Palladium, zu Base wird vorzugsweise so gewählt, daß pro Grammatom Platinmetall, z.B. Palladium, 0,1 bis 500, bevorzugt 0,3 bis 200 besonders bevorzugt 0,9 bis 130 Äquivalente Base eingesetzt werden.

Die für das erfindungsgemäße Verfahren geeigneten Platinmetall-Katalysatoren bestehen aus mindestens einem Edelmetall der Gruppe VIII, vorzugsweise Palladium. Es kann bei dem erfindungsgemäßen Verfahren in verschiedener Form zugegeben werden. Palladium kann in metallischer Form oder bevorzugt in Form von PalladiumVerbindungen der Oxidationsstufen 0 und +2, wie beispielsweise Palladium(II)-acetylacetonat, -halogenide, -carboxylate von C₂-C₁₈-Carbonsäuren, -nitrat, -oxide oder Palladiumkomplexe, die beispielsweise Olefine, Amine, Phosphorverbindungen und Halogenide enthalten können, eingesetzt werden. Besonders bevorzugt sind Palladiumbromid und Palladiumacetylacetonat.

Die Menge an Platinmetall-Katalysator ist im erfindungsgemäßen Verfahren nicht beschränkt. Bevorzugt wird so viel Katalysator zugesetzt, daß die Konzentration des Metalls im Reaktionsansatz 1 - 3000 ppm beträgt, besonders bevorzugt sind Konzentrationen von 5- 500 ppm.

Als Cokatalysator für das erfindungsgemäße Verfahren wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew), gegebenenfalls auch Mischungen davon, verwendet, wobei das Metall in verschiedenen Oxidationsstufen eingesetzt werden kann. Bevorzugt werden Mn, Cu, Co, V, Zn, Ce und Mo eingesetzt. Ohne das erfindungsgemäße Verfahren einzuschränken, seien Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₆-Carbonsäuren, Diketonate oder Nitrate sowie als Komplexverbindungen eingesetzt werden, die beispielsweise Kohlenmonoxid, Olefine, Amine, Phosphorverbindungen und Halogenide enthalten können. Besonders bevorzugt werden Mn, Cu, Mo und Ce eingesetzt. Ganz besonders bevorzugt werden Manganverbindungen im erfindungsgemäßen Verfahren verwendet, besonders bevorzugt Mangan(II)-und Mangan(III)komplexe, ganz besonders bevorzugt Mangan(II)acetylacetonat bzw. Mangan(III)-acetylacetonat.

Der Cokatalysator, der auch in situ gebildet werden kann, wird in einer solchen Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den im Rahmen der vorliegenden Erfindung eingesetzten quatemären Salzen kann es sich beispielsweise um mit organischen Resten substituierte Ammonium-, Guanidinium-, Phosphonium- oder Sulfoniumsalze, gegebenenfalls auch Mischungen davon, handeln. Geeignet für den Einsatz in das erfindungsgemäße Verfahren sind Ammonium-, Guanidinium-, Phosphonium- und Sulfoniumsalze, die als organische Reste C₆bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat tragen. Bevorzugt werden in das erfindungsgemäße Verfahren Ammonium- und Phosphoniumsalze eingesetzt, die als organische Reste C₆- bis C₁₀-Aryl-, C₇- bis C₁₂-Aralkyl- und/oder C₁- bis C₂₀-Alkyl-Reste und als Anion ein Halogenid tragen, besonders bevorzugt ist Tetrabutylammoniumbromid und Tetrabutylphosphoniumbromid. Die Menge eines solchen quaternären Salzes kann beispielsweise 0,1 - 20 Gew.-%, bezogen auf das Gewicht des Reaktionsgemisches, betragen. Vorzugsweise beträgt diese Menge 0,5 - 15 Gew.-%, besonders bevorzugt 1 - 5 Gew.-%.

Bei Verwendung einer Base wie Tetrabutylammonium-tert.-butylat, welche ein quaternäres Kation enthält, kann die Menge an zugesetztem quaternären Salz wie Tetrabutylammoniumbromid entsprechend verringert werden. Gegebenenfalls kann man die Gesamtmenge des Anions des zugesetzten quaternären Salzes auch durch andere Salze dieses Anions wie Kaliumbromid ausgleichen.

In einer weiteren Ausführungsform werden statt des homogenen Katalysatorsystems heterogene Katalysatoren, bei denen das Platinmetall oder das Platinmetall und der Cokatalysator auf einem heterogenen Träger aufgebracht sind, als Pulver oder Formkörper eingesetzt. Die übrigen Komponenten des Katalysatorsystems, wie die Base, die quaternäre Verbindung und gegebenenfalls der Cokatalysator, sind weiterhin in der Reaktionslösung homogen gelöst. Die Menge des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der oben genannten Art eingesetzt.

Die Menge des Cokatalysators am Gesamtgewicht des heterogenen Katalysators beträgt 0,01 bis 15 Gew.-%, bevorzugt 0,05 bis 10 Gew.-% gerechnet als Metall.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch sowie Eisen- und Kobaltoxide, Nickel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Trägerkatalysator-Pulver bezogen auf die eingesetzte Menge an aromatischer Hydroxyverbindung, verwendet.

In bevorzugten Ausführungsformen wird der heterogene Trägerkatalysator ortsfest in Rührbehältem, einer Blasensäule, einem Rieselphasenreaktor oder Kaskaden dieser Reaktoren eingesetzt. Eine Abtrennung des Trägerkatalysators entfällt dann völlig.

Als Reaktoren für das erfindungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß zu verwenden Rührbehälter mit dafür geeigneten Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können in dem erfindungsgemäßen Verfahren folgende Typen, eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren (Chem. Ing. Tech. 51 (1979) Nr. 3, S. 208-216; W.-D. Deckwer, Reaktionstechnik in Blasensäulen, Otto Salle Verlag 1985).

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfindungsgemäße Verfahren kann in verschiedenen Ausführungsvarianten ausgeübt werden. Eine Möglichkeit besteht in der diskontinuierlichen Durchführung. Dabei werden CO und Sauerstoff entweder durch einen Begasungsrührer wie im Falle eines Rührkessels oder durch andere bekannte Gasverteilungsorgane in die Reaktionsmischung geleitet. Nach Erreichen des optimalen Umsatzes wird das Reaktionsgemisch aus dem Reaktor entfernt oder gegebenenfalls im Reaktor aufgearbeitet. Im Falle der Verwendung pulverförmigen Trägerkatalysatoren können diese aus der Reaktionsmischung z.B. durch Filtration, Sedimentation oder Zentrifugieren abgetrennt werden.

In diskontinuierlichen Versuchen verwendete Trägerkatalysatoren können bei gleichen Einsatzstoffen gegebenenfalls ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch gegebenenfalls regeneriert werden.

### Beispiele

### Beispiel 1

In einem 600 ml Planschlifftopf mit Heizmantel, Begasungsrührer, Kühler und nachgeschalteter Kühlfalle wurden 0,3 mmol Palladiumbromid, 3,0 mmol Mangan(II)-acetylacetonat und 30,0 mmol Tetrabutylammoniumbromid in 350 ml Chlorbenzol vorgelegt und eine Stunde lang bei 80 °C Kohlenmonoxid (3l/h) zum Lösen des Katalysators durchgeleitet. Dann wurden 21,0 g Bisphenol A und 5,2 mmol Kalium-tert.-butylat zugegeben, auf 80 °C erhitzt und unter Einleitung von 180 Nl/h eines Gasgemisches aus Kohlenmonoxid und Sauerstoff (95:5 Vol.-%) unter Normaldruck die Reaktion gestartet. Nach 6 h Reaktionszeit werden innerhalb von 8 Stunden jeweils 0,8 g Bisphenol A pro Stunde als Feststoff zugegeben. Nach insgesamt 20 h sind laut HPLC-Analyse 95 % der Gesamtmenge an Bisphenol A umgesetzt. Das Produktspektrum enthält die Oligomeren gemäß Struktur (I) mit n = 1-7. In der Kühlfalle befand sich ca. 10-15 % der eingesetzten Menge an Chlorbenzol; die stets auftretende Phasentrennung ermöglicht das einfache Entfernen des gebildeten Wassers aus dem Kondensat. Nach Versuchsende hatte die Reaktionsmischung einen Wasserrestgehalt von weniger als 250 ppm.

### Beispiel 2

In einem 250 ml Autoklaven mit Begasungsrührer, Kühler und nachgeschalteter Kühlfalle wurden 0,15 mmol Palladiumbromid, 11 mmol Tetrabutylammoniumbromid und 8 g Bisphenol A in 90 ml Chlorbenzol vorgelegt und 30 min bei 90 °C unter Einleitung von Kohlenmonoxid (3 l/h) gelöst. Anschließend wurden 1,1 mmol Mangan(III)acetylacetonat und 5,2 mmol Kalium-tert.-butylat mit 10 ml Chlorbenzol zugegeben, und unter Einleitung von 80 Nl/h eines Gasgemisches aus Kohlenmonoxid und Sauerstoff (95:5 Vol%) bei 3 bar Gesamtdruck und 110 °C die Reaktion gestartet. Nach 6h wird die Reaktionslösung mit 100 ml Methylenchlorid verdünnt. Die HPLC-Analysen ergaben, daß insgesamt 91 % an Bisphenol A umgesetzt wurden und das Produktspektrum die Oligomeren gemäß Struktur (I) mit n = 1 - 9 enthält. In der Kühlfalle waren 12 g einer Clorbenzol/Wasser-Suspension kondensiert. Nach Versuchsende hatte die Reaktionsmischung einen Wasserrestgehalt von unter 250 ppm.

### Beispiel 3 (Vergleich)

In einem 250 ml Autoklaven mit Begasungsrührer, Kühler und nachgeschalteter Kühlfalle wurden 0,15 mmol Palladiumbromid, 11 mmol Tetrabutylammoniumbromid und 8 g Bisphenol A in 90 ml Chlorbenzol vorgelegt und 30 min bei 90 °C unter Einleitung von Kohlenmonoxid (3 l/h) gelöst. Anschließend wurden 1,1 mmol Mangan(III)acetylacetonat und 5,2 mmol Kalium-tert.-butylat mit 10 ml Chlorbenzol zugegeben, und unter Einleitung von 80 Nl/h eines Gasgemisches aus Kohlenmonoxid und Sauerstoff (95:5 Vol%) bei 7 bar Gesamtdruck und 110 °C die Reaktion gestartet. Nach 6h wird die Reaktionslösung mit 100 ml Methylenchlorid verdünnt. Die HPLC-Analysen ergaben, daß insgesamt 38 % der Gesamtmenge an Bisphenol A umgesetzt wurden und das Produktspektrum die Oligomeren gemäß Struktur (I) mit n = 1 - 5 enthält. In der Kühlfalle waren weniger als. 3 g Wasser kondensiert. Nach Versuchsende hatte die Reaktionsmischung einen Wasserrestgehalt von mehr als 600 ppm. Die verwendete Einstellung von Druck und Temperatur entsprechen reinen Strippbedingungen, da das Azeotrop praktisch nicht aus dem Reaktionsgemisch entfernt wurde. Der direkte Vergleich mit Beispiel 2 belegt die Effizienz der Entfernung des Azeotrops aus dem Reaktionsgemisch.

## Patentansprüche

1. Verfahren zur Herstellung eines aromatischen Oligocarbonats der Formel
HO-[A₁-Y-A₂-O-CO-O]ₙ-A₁-Y-A₂-OH (I),
wobei
A₁, A₂ voneinander unabhängig divalente carbocyclische oder heterocyclische aromatische Gruppen repräsentieren,
Y eine divalente Gruppe wie Alkylen, Alkyliden, Cycloalkylen, Cycloalkyliden, -S-, -O-, -SO₂-, -SO- und -CO- oder eine Einfachbindung bedeutet und
n Zahlen von 1 bis 75 annehmen kann,
durch Umsetzung einer aromatischen Hydroxyverbindung der Formel
HO-A₁-Y-A₂-OH (II),
worin A₁, A₂ und Y die oben angegebenen Bedeutungen haben,
mit Kohlenmonoxid und Sauerstoff in Gegenwart eines Platinmetall-Katalysators, eines Cokatalysators, einer Base, und gegebenenfalls eines quaternären Salzes, sowie eines inerten organischen Lösungsmittels, **dadurch gekennzeichnet, dass** das inerte organische Lösungsmittel unter den Reaktionsbedingungen ein Azeotrop mit dem bei der Reaktion entstehenden Wasser bildet, und dieses Azeotrop aus dem Reaktionsgemisch entfernt wird.

2. Verfahren gemäß Anspruch 1, **dadurch gekennzeichnet, dass** die Entfernung des Azeotrops aus dem Reaktionsgemisch durch überschüssiges Reaktionsgas unterstützt wird.

3. Verfahren gemäß Anspruch 1 oder 2, **dadurch gekennzeichnet, dass** als inertes organisches Lösungsmittel aromatische Lösemittel eingesetzt werden.

4. Verfahren gemäß Anspruch 3, **dadurch gekennzeichnet, dass** als inertes organisches Lösungsmittel Chlorbenzol eingesetzt wird.

5. Verfahren gemäß Anspruch 1 oder 2, **dadurch gekennzeichnet, dass** als inertes organisches Lösungsmittel halogenierte Kohlenwasserstoffe eingesetzt werden.

6. Verfahren nach Anspruch 5, **dadurch gekennzeichnet, dass** als Lösungsmittel 1,2-Dichlorethan verwendet wird.

7. Verfahren nach einem der Ansprüche 1 bis 6, **dadurch gekennzeichnet, dass** als Basen Alkalisalze bzw. quaternäre Salze schwacher Säuren verwendet werden.

8. Verfahren nach Anspruch 7, **dadurch gekennzeichnet, dass** als Base das Monooder das Dialkalisalz des eingesetzten Diphenols der allg. Formel (II) oder eine Mischung davon verwendet wird.

9. Verfahren nach Anspruch 7, **dadurch gekennzeichnet, dass** als Base Kaliumtert.-butylat verwendet wird.

10. Verfahren nach einem der Ansprüche 1 bis 6, **dadurch gekennzeichnet, dass** als Basen Alkalihydroxide verwendet werden.

## Claims

1. Process for the preparation of an aromatic oligocarbonate of the formula
HO-[A₁-Y-A₂-O-CO-O]ₙ-A₁-Y-A₂-OH (I),
wherein
A₁,A₂ independently of one another represent divalent carbocyclic or heterocyclic aromatic groups,
Y denotes a divalent group, such as alkylene, alkylidene, cycloalkylene, cycloalkylidene, -S-, -O-, -SO₂-, -SO- and -CO- or a single bond and
n can assume numbers from 1 to 75,
by reaction of an aromatic hydroxy compound of the formula
HO-A₁-Y-A₂-OH (II),
wherein A₁, A₂ and Y have the abovementioned meanings,
with carbon monoxide and oxygen in the presence of a platinum metal catalyst, a cocatalyst, a base and optionally a quaternary salt, as well as an inert organic solvent, **characterised in that** under the reaction conditions, the inert organic solvent forms an azeotrope with the water formed during the reaction, and this azeotrope is removed from the reaction mixture.

2. Process according to claim 1, **characterised in that** the removal of the azeotrope from the reaction mixture is assisted by excess reaction gas.

3. Process according to claim 1 or 2, **characterised in that** aromatic solvents are employed as the inert organic solvent.

4. Process according to claim 3, **characterised in that** chlorobenzene is employed as the inert organic solvent.

5. Process according to claim 1 or 2, **characterised in that** halogenated hydrocarbons are employed as the inert organic solvent.

6. Process according to claim 5, **characterised in that** 1,2-dichloroethane is used as the solvent.

7. Process according to one of claims 1 to 6, **characterised in that** alkali metal salts or quaternary salts of weak acids are used as the bases.

8. Process according to claim 7, **characterised in that** the mono- or the dialkali metal salt of the diphenol of the general formula (II) employed or a mixture thereof is used as the base.

9. Process according to claim 7, **characterised in that** potassium tert-butylate is used as the base.

10. Process according to one of claims 1 to 6, **characterised in that** alkali metal hydroxides are used as the bases.

## Revendications

1. Procédé de préparation d'un oligocarbonate aromatique de la formule :
HO-[A₁-Y-A₂-O-CO-O]ₙ-A₂-Y-A₂-OH (I)
où
A₁, A₂ représentent indépendamment l'un de l'autre, un groupe bivalent aromatique carbocyclique ou hétérocyclique ;
Y représente un radical bivalent comme les radicaux alcoylène, alcoylidène, cycloalcoylène, cycloalcoylidène, -S-, -O-, -SO₂-, -SO- et -CO- ou une simple liaison, et
n peut prendre les valeurs de 1 à 75,
par réaction d'un composé hydroxylé aromatique de la formule :
HO-A₁-Y-A₂-OH (II) (II)
où A₁, A₂ et Y ont les significations indiquées ci-dessus,
avec du monoxyde de carbone et de l'oxygène en présence d'un catalyseur au platine métallique, un cocatalyseur, une base et le cas échéant, un sel quaternaire, ainsi qu'un solvant organique inerte, **caractérisé en ce que** le solvant organique inerte forme dans les conditions de réaction, un azéotrope avec l'eau formée par la réaction et cet azéotrope est éliminé du mélange réactionnel.

2. Procédé selon la revendication 1, **caractérisé en ce que** l'élimination de l'azéotrope du mélange réactionnel est facilitée par du gaz de réaction en excès.

3. Procédé selon la revendication 1 ou 2, **caractérisé en ce que** l'on met en oeuvre comme solvant organique inerte, un solvant aromatique.

4. Procédé selon la revendication 3, **caractérisé en ce que** l'on met en oeuvre le chlorobenzène comme solvant organique inerte.

5. Procédé selon la revendication 1 ou 2, **caractérisé en ce que** l'on met en oeuvre comme solvant organique inerte, un hydrocarbure halogéné.

6. Procédé selon la revendication 5, **caractérisé en ce que** l'on utilise le 1,2-dichloroéthane comme solvant.

7. Procédé selon l'une quelconque des revendications 1 à 6, **caractérisé en ce que** l'on utilise un sel alcalin ou un sel quaternaire d'acide faible comme base.

8. Procédé selon la revendication 7, **caractérisé en ce que** l'on utilise comme base, le mono- ou di-sel alcalin du diphénol mis en oeuvre de la formule générale (II) ou un mélange de ceux-ci.

9. Procédé selon la revendication 7, **caractérisé en ce que** l'on utilise le t-butylate de potassium comme base.

10. Procédé selon l'une quelconque des revendications 1 à 6, **caractérisé en ce que** l'on utilise un hydroxyde alcalin comme base.
